# DEMANDE DE BREVET EUROPEEN

(11) **EP 3 196 204 A1**
(43) Date de publication de la demande: **26.07.2017**
(21) Numéro de dépôt: 17154216.0
(22) Date de dépôt: 04.02.2014
(51) Int. Cl.: C07H 1/00, C07H 15/04, C07D 493/04, C07D 307/20, A61Q 1/14, A61Q 17/04, A61Q 19/00, A61Q 19/04, A61Q 19/08, A61Q 19/10, A61K 8/60

(54) **NOUVELLE COMPOSITION DE POLYOLS-GLYCOSIDES**

(30) Priorité: 26.03.2013 FR 1352693
(62) Demande divisionnaire de: 14708614.4
(71) Demandeur: Societe D Exploitation De Produits Pour Les Industries Chimiques Seppic, 75007 Paris (FR)
(72) Inventeur: BENATTAR, André, 81100 CASTRES (FR); BONNARDEL, Andrey, 81100 CASTRES (FR); GUILBOT, Jerôme, 81100 CASTRES (FR); KERVERDO, Sébastien, 94300 Vincennes (FR); ROLLAND, Hervé, 81100 CASTRES (FR); TABACCHI, Guy, 75012 PARIS (FR)
(74) Mandataire: Conan, Philippe Claude

(57) **Abrégé**

Composition (C₂) comprenant pour 100% de sa masse :
- De 1 % à 70% massique d'un polyol de formule (A₁) :

HO-CH₂-(CHOH)ₙ-CH₂-OH (A₁)

dans laquelle n est un nombre entier égal à 2, 3 ou 4 ;
- De 25% à 98,9% massique d'une composition (C₁) représentée par la formule (I) :

HO-CH₂-(CHOH)ₙ-CH₂-O-(G)ₓ-H (I),

Dans laquelle G représente le reste d'un sucre réducteur, n est un nombre entier égal à 2, 3 ou 4 et x, qui indique le degré moyen de polymérisation dudit reste G, représente un nombre décimal supérieur à 1 et inférieur ou égal à 5 ;
- De 0,1% à 5% massique d'un composé (B) ou d'un mélange de composés (B) choisis parmi :
- Le composé de formule (B₁₁) : 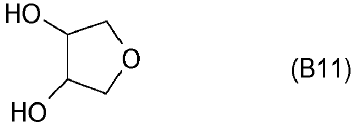 - Le composé de formule (B₁₂) : 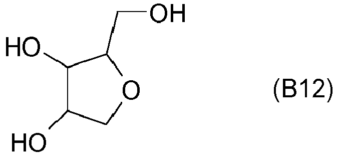 - Le composé de formule (B₁₃) : 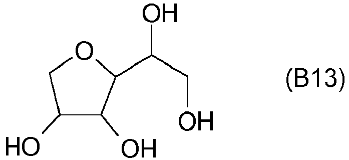 - Et le composé de formule (B₁₄) : 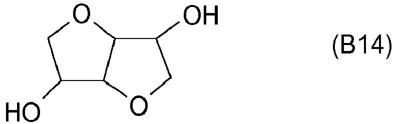 Son utilisation en cosmétique.

## Description

L'invention concerne un nouveau procédé de préparation de polyols-glycosides, ainsi que de nouvelles compositions à base de polyols-glycosides issus d'un tel procédé.

L'invention trouve principalement application dans les domaines de la cosmétique, de la dermocosmétique, de la pharmacie et de la dermopharmacie, mais également dans le domaine de l'alimentaire comme par exemple comme agent édulcorant et/ou agent édulcorant de charge, dans le domaine de l'industrie textile par exemple pour le traitement de fibres textiles synthétiques ou naturelles tissées ou tricotées, ou encore dans le domaine de l'industrie papetière par exemple pour la fabrication de papier à usage sanitaire ou domestique.

Les polyols-glycosides peuvent être préparés en faisant réagir un polyoside, comme l'amidon, avec un polyol, comme un composé aliphatique linéaire ou ramifié, comportant de deux à vingt atomes de carbone, et comportant au moins deux groupes hydroxyle. Ces procédés sont généralement mis en oeuvre dans des conditions de pressions élevées, et à une température généralement supérieure ou égale à 120°C, en présence d'au moins un catalyseur acide. Le polyoside est hydrolysé en oligo-oside et en mono-oside qui réagissent avec le polyol selon une réaction d'acétalisation, pour former les polyols-glycosides.

Le brevet américain publié sous le numéro US 3,346,558 divulgue plus particulièrement un procédé continu de préparation de polyol-glycosides mettant en oeuvre de l'amidon et un polyol aliphatique comportant de deux à six groupes hydroxyle, à une température comprise entre 170°C et 300°C et sous une pression supérieure à la pression de vapeur du mélange réactionnel, en présence d'un catalyseur acide et plus particulièrement d'un acide sulfonique ou d'un acide de Lewis.

Ces procédés ont cependant des conditions opératoires très consommatrices d'énergie, les polyols-glycosides obtenus présente une structure glycosidique mal maitrisée et leur couleur souvent foncée les rend impropres à des utilisations pour préparer de compositions cosmétiques et/ou pharmaceutiques.

Les polyols-glycosides peuvent aussi être préparés en faisant réagir un sucre réducteur à structure mieux définie, comme un monosaccharide, avec un polyol tel que décrit ci-dessus, selon une réaction d'acétalisation dans des conditions de température modérées, généralement entre 70°C et 130°C, à pression atmosphérique ou sous pression réduite, généralement entre 3x10⁴Pa (300mbar) et 2x10³Pa (20 mbar), et en présence d'un système catalytique acide généralement choisi parmi les acides forts minéraux, les acides forts organiques, les acides carboxyliques, les acides de Lewis et les résines échangeuses d'ions.

Le brevet américain publié sous le numéro US 4,024,290 divulgue la préparation de glucosyl sorbitol par réaction entre le glucose et le sorbitol, à une température de 160°C, sous pression réduite, en présence d'une résine échangeuse d'ions de type acide. Un tel procédé permet d'obtenir majoritairement le glucosyl sorbitol, produit de la réaction d'acétalisation du sorbitol avec le glucose, ainsi que du sorbitol résiduel et des sous-produits.

Le brevet américain publié sous le numéro US 3,772,269 divulgue que les réactions d'acétalisation de glycols aliphatiques avec des sucres réducteurs, sont réalisées en présence d'acides forts minéraux, comme l'acide chlorhydrique, l'acide sulfurique, l'acide phosphorique, l'acide phosphoreux, ou d'acides de Lewis, comme le trifluorure de bore, d'acides forts organiques, comme l'acide para-toluène sulfonique, ou de résines échangeuses d'ions ; l'acide sulfurique étant considéré comme le catalyseur préféré de fait de sa facilité d'élimination à l'issue de la réaction d'acétalisation.

L'utilisation de tels systèmes catalytiques présentent cependant le double inconvénient de favoriser la formation en des quantités non désirées de polyglucoses et d'atteindre des produits de réaction fortement colorés.

Pour pallier à ces deux problèmes, l'homme du métier peut mettre en oeuvre une réaction d'acétalisation par réaction directe d'un sucre réducteur et d'un excès d'alcool, en présence d'un système catalytique acide, suivie par une étape de distillation de l'alcool gras résiduel, et si nécessaire suivie par une étape de décoloration par ajout d'eau oxygénée dans des conditions de pH prédéterminées.

Une autre approche complémentaire consiste à mettre en oeuvre des systèmes catalytiques spécifiques. La demande de brevet européen publiée sous le numéro EP 0 570 056 A1 décrit la préparation d'alkyl polyglucosides par acétalisation du glucose avec des alcools gras, en présence d'un système catalytique formé par un acide organique fort, sélectionné dans le groupe constitué par des acides alkyl benzènesulfoniques et des acides alcanesulfoniques secondaires ou tertiaires, et une base faible organique ayant une valeur de Ka comprise entre 10⁻⁸ et 10⁻¹, sélectionnée parmi la pyridine, les picolines, les lutidines, les collidines, les quinoléines, l'isoquinoléine, la quinaldine, la pyrazine, le ptéridine et le N,N,N'N'-(tétraméthyl) urée.

La demande de brevet européen publiée sous le numéro EP 0 077 167 A décrit un procédé d'obtention d'alkyl glycosides à partir de monosaccharides et d'alcools linéaires ou branchés, comportant au moins 10 atomes de carbone, en présence d'un système catalytique acide constitué d'un catalyseur acide et d'un agent réducteur. Les catalyseurs acides mis en oeuvre dans un tel procédé sont les catalyseurs acides connus de l'homme du métier, comme par exemple l'acide sulfurique, l'acide chlorhydrique, l'acide nitrique, les acides sulphoniques, comme par exemple l'acide méthanesulfonique, l'acide para-toluènesulfonique, l'acide trifluoro méthanesulfonique, et les résines échangeuses d'ions acides forts ; les agents réducteurs mis en oeuvre dans un tel procédé sont choisis parmi l'acide phosphoreux, l'acide hypophosphoreux, l'acide sulfureux, l'acide hyposulfureux, l'acide nitreux et l'acide hyponitreux.

Cet état de la technique associé à la préparation d'alkylpolyglycosides, est transposé communément pour la préparation de polyols-glycosides. Ainsi, la demande internationale publiée sous le numéro WO 03/094864 divulgue la préparation de polyol-glycosides par acétalisation d'un sucre réducteur et d'un polyol de formule (A₁) :

HO-CH₂-(CHOH)ₙ-CH₂-OH (A₁)

dans laquelle n est un nombre entier égal à 2, 3 ou 4, en présence d'un système catalytique acide choisi parmi l'acide sulfurique, l'acide chlorhydrique, l'acide phosphorique, l'acide nitrique, l'acide hypophosphoreux, l'acide méthanesulfonique, l'acide para-toluènesulfonique, l'acide trifluorométhanesulfonique et les résines échanges d'ions acides, et plus particulièrement la préparation de xylityl-glucoside et d'érythrityl-glucoside par la réaction du glucose avec les polyols correspondant en présence d'un système catalytique acide composé d'acide sulfurique.

Si le choix de tels systèmes catalytiques permet d'accroitre la conversion du polyol en polyol-glucoside, on observe cependant la formation de sous-produits issus de la déshydratation du polyol de formule (A₁) en milieu acide conduisant à la formation de dérivés cycliques dudit polyol de formule (A₁).

Ainsi :
- Lorsque le polyol de formule (A₁) est l'érythritol (n = 2), il se déshydrate en milieu acide en 3,4-dihydroxy tétrahydrofuranne de formule (B₁₁) : 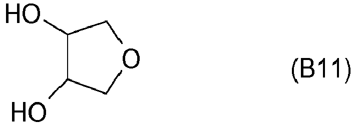
- Lorsque le polyol de formule (A₁) est le xylitol (n = 3), il se déshydrate en milieu acide 3,4-dihydroxy 2-(hydroxyméthyl) tétrahydrofuranne de formule (B₁₂) (ou 1,4-anhydroxylitol) : 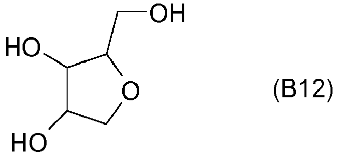
- Lorsque le polyol de formule (A₁) est le sorbitol (n = 4), il se déshydrate en milieu acide en 2-(1,2-dihydroxy éthyl) 3,4-dihydroxy tétrahydrofuranne de formule (B₁₃) (ou 1,4-anhydrosorbitol) et en 1,5-dioxa bicyclo[3,3,0]octan-3,7-diol de formule (B₁₄) (ou isosorbide) :

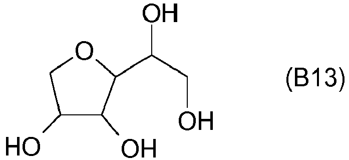

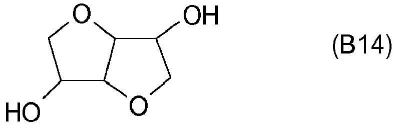

La formation de ces sous-produits de déshydratation des polyols de formule (A₁), concurrence la réaction d'acétalisation, et ne permet pas d'obtenir un rendement de formation de polyols-glycosides satisfaisant.

Les inventeurs ont donc cherché à développer un nouveau procédé, qui minimise de façon importante la formation des sous-produits de déshydratation des polyols de formule (A₁).

Ainsi, selon un premier aspect, l'invention a pour objet un procédé de préparation d'une composition (C₁) représentée par la formule (I) :

HO-CH₂-(CHOH)ₙ-CH₂-O-(G)ₓ-H (I),

Dans laquelle G représente le reste d'un sucre réducteur, n est un nombre entier égal à 2, 3 ou 4 et x, qui indique le degré moyen de polymérisation dudit reste G, représente un nombre décimal supérieur à 1 et inférieur ou égal à 5, caractérisé en ce que ledit procédé comprend au moins une étape a) de réaction d'un polyol de formule (A₁) :

HO-CH₂-(CHOH)ₙ-CH₂-OH (A₁),

Dans laquelle n est un nombre entier égal à 2, 3 ou 4, avec un sucre réducteur de formule (II) :

HO-G-H (II)

Dans laquelle G représente le reste d'un sucre réducteur, en présence d'un catalyseur acide (Cₐ), et en ce que ledit catalyseur acide (Cₐ) est choisi parmi l'acide hypophosphoreux, l'acide phosphorique et l'acide polyphosphorique.

Par acide polyphosphorique, on désigne les composés de formule (Cₐ₁) :

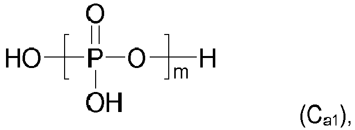

Dans laquelle m représente un nombre décimal supérieur à 1 et inférieur ou égal à 15, plus particulièrement supérieur ou égal à 2 et inférieur ou égal à 5, et encore plus particulièrement supérieur ou égal à 2 et inférieur ou égal à 3.

Par sucre réducteur, on désigne dans les formules (I) et (II) telles que définies précédemment, les dérivés saccharidiques qui ne présentent pas dans leurs structures de liaison glycosidique établie entre un carbone anomérique et l'oxygène d'un groupement acétal tels qu'ils sont définis dans l'ouvrage de référence : "Biochemistry", Daniel Voet/Judith G. Voet, p. 250, John Wyley & Sons, 1990. La structure oligomérique (G)ₓ peut se présenter sous toutes formes d'isoméries, qu'il s'agisse d'isomérie optique, d'isomérie géométrique ou d'isomérie de position ; elle peut aussi représenter un mélange d'isomères.

Par HO-CH₂-(CHOH)ₙ-CH₂-O-(G)ₓ-H, on signifie que ladite composition (C₁) consiste essentiellement en un mélange de composés représentés par les formules (I₁), (I₂), (I₃), (I₄) et (I₅) :

HO-CH₂-(CHOH)ₙ-CH₂-O-(G)₁-H (I₁),

HO-CH₂-(CHOH)ₙ-CH₂-O-(G)₂-H (I₂),

HO-CH₂-(CHOH)ₙ-CH₂-O-(G)₃-H (I₃),

HO-CH₂-(CHOH)ₙ-CH₂-O-(G)₄-H (I₄),

HO-CH₂-(CHOH)ₙ-CH₂-O-(G)₅-H (I₅),

Dans les proportions molaires respectives a₁, a₂, a₃, a₄ et a₅, telles que la somme a₁ + a₂ + a₃ + a₄ + a₅ est égale à 1 et que chacune des proportions a₁, a₂, a₃, a₄ et a₅ est supérieure ou égale à zéro et inférieure ou égale à un.

Par essentiellement, on indique dans la définition qui précède, que la présence d'un ou de plusieurs composés de formule (I_{w}) avec w supérieur à 5 n'est pas exclue au sein de la composition (C₁), mais que si présence il y a, elle l'est en des proportions minimes qui n'entrainent aucune modification substantielle des propriétés de ladite composition (C₁).

Dans la formule (I) telle que définie ci-dessus, le groupe :

HO-CH₂-(CHOH)ₙ-CH₂-O-,

Est lié à (G)ₓ par le carbone anomérique du reste saccharide, de manière à former une fonction acétal.

L'étape a) du procédé tel que défini précédemment, est mise en oeuvre dans un réacteur, par dispersion du sucre réducteur de formule (II) sur le polyol de formule (A₁) préalablement amené à une température (T₁) supérieure d'au moins 5°C à sa température de fusion, sous agitation mécanique. Lorsque ledit sucre réducteur de formule (II) est dispersé de façon homogène sur ledit polyol de formule (A₁), ledit catalyseur acide (Cₐ) est ajouté, puis le milieu réactionnel ainsi préparé est maintenu pendant une durée comprise entre 3 heures et 7 heures, sous un vide partiel compris entre 300 mbar (3.10⁴ Pa) et 20 mbar (2.10³ Pa), à une température (T₂) supérieure d'au moins 5°C à la température de fusion du polyol de formule (A₁).

Lorsque le polyol de formule (A₁) est le xylitol ou le sorbitol, la température (T₁) est supérieure ou égale à 95°C et inférieure ou égale à 130°C et plus particulièrement supérieure ou égale à 95°C et inférieure ou égale à 115°C tandis que la température (T₂) est supérieure ou égale à 95°C et inférieure ou égale à 130°C et plus particulièrement supérieure ou égale à 105°C et inférieure ou égale à 120°C.

Lorsque le polyol de formule (A₁) est l'érythritol, les températures (T₁) et(T₂), identiques ou différentes sont supérieures ou égales à 120°C et inférieures ou égales à 135°C et plus particulièrement inférieures ou égale à 130°C.

L'étape a) du procédé tel que défini précédemment, peut être complétée, si nécessaire ou si désirée, par des opérations ultérieures de neutralisation, par exemple à la soude ou à la potasse, et/ou de filtration, et/ou de décoloration, et/ou d'élimination du polyol résiduel par exemple par extraction sélective au moyen d'un milieu solvant adapté.

Selon un aspect particulier de la présente invention, dans la définition des composés de formules (I) et (II), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose.

Selon un autre aspect particulier de la présente invention, dans la définition du polyol de formule (A₁), n est un nombre entier égal à 2.

Selon un autre aspect particulier de la présente invention, dans la définition du polyol de formule (A₁), n est un nombre entier égal à 3.

Selon un autre aspect particulier de la présente invention, dans la définition du polyol de formule (A₁), n est un nombre entier égal à 4.

Selon un aspect particulier, la présente invention a pour objet un procédé tel que défini précédemment, pour lequel dans la définition des composés de formule (I) et des composés de formule (II), G représente le reste d'un sucre réducteur choisi parmi les restes du glucose, du xylose et de l'arabinose.

Selon un aspect encore plus particulier de la présente invention, la présente invention a pour objet un procédé tel que défini précédemment, caractérisé en ce que dans la formule (I), x représente un nombre décimal supérieur ou égal à 1,05 et inférieur ou égal à 3, plus particulièrement supérieur ou égal à 1,15 et inférieur ou égal à 2,5.

Selon un autre aspect particulier, la présente invention a pour objet un procédé tel que défini précédemment, caractérisé en ce que dans l'étape a) le rapport molaire, sucre réducteur de formule (II) sur polyol de formule (A₁), est supérieur ou égal à 1/6 et inférieur ou égal à 4/1, plus particulièrement supérieur ou égal 1/3 et inférieur ou égal à 4/1, et encore plus particulièrement supérieur ou égal à 1/3 et inférieur ou égal à 2/1.

Selon un autre aspect particulier, la présente invention a pour objet un procédé tel que défini précédemment, caractérisé en ce que, à l'étape a), la proportion massique en catalyseur acide (Cₐ), est supérieure ou égale à 0,05% et inférieure ou égale à 2% pour 100% de la somme des masses de sucre réducteur de formule (II) et de polyol de formule (A₁) ; plus particulièrement supérieure ou égale à 0,1 % et inférieure ou égale à 1% pour 100% de la somme des masses de sucre réducteur de formule (II) et de polyol de formule (A₁) ; et encore plus particulièrement supérieure ou égale à 0,2 % et inférieure ou égale à 1%, pour 100% de la somme des masses de sucre réducteur de formule (II) et de polyol de formule (A₁).

Selon un autre aspect particulier, la présente invention a pour objet un procédé tel que défini précédemment, caractérisé en ce que, à l'étape a), le catalyseur acide (Cₐ) mis en oeuvre est l'acide hypophosphoreux.

Selon un autre aspect particulier, la présente invention a pour objet un procédé tel que défini précédemment, caractérisé en ce que, à l'étape a), le catalyseur acide (Cₐ) mis en oeuvre est l'acide phosphorique.

L'invention a aussi pour objet une composition (C₂) comprenant pour 100% de sa masse :
- De 1% à 70% massique d'un polyol de formule (A₁) :

   HO-CH₂-(CHOH)ₙ-CH₂-OH (A₁),

Dans laquelle n est un nombre entier égal à 2, 3 ou 4 ;
- De 25% à 98,9% massique d'une composition (C₁) représentée par la formule (I) :

   HO-CH₂-(CHOH)ₙ-CH₂-O-(G)ₓ-H (I),

Dans laquelle G représente le reste d'un sucre réducteur, n est un nombre entier égal à 2, 3 ou 4 et x, qui indique le degré moyen de polymérisation dudit reste G, représente un nombre décimal supérieur à 1 et inférieur ou égal à 5 ;
- De 0,1% à 5% massique d'un composé (B) ou d'un mélange de composés (B) choisis parmi :
   - Le composé de formule (B₁₁) : 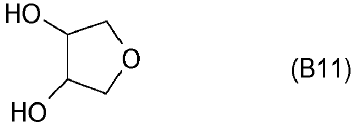
   - Le composé de formule (B₁₂) : 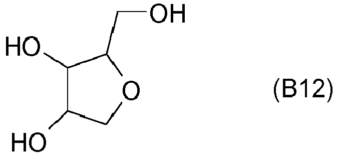
   - Le composé de formule (B₁₃) : 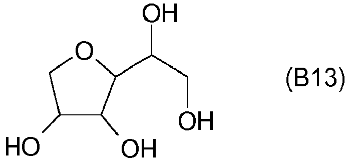
   - Et le composé de formule (B₁₄) :

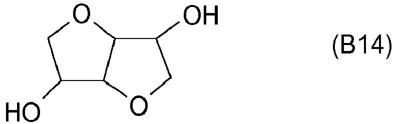

Selon un aspect particulier de la présente invention, la composition (C₂) telle que définie ci-dessus, comprend pour 100% de sa masse :
- De 5% à 65% massique, plus particulièrement de 10% à 60% massique d'au moins un polyol de formule (A₁),
- De 31 % à 94,5% massique, plus particulièrement de 37% à 89,5% massique d'au moins une composition (C₁),
- De 0,5% à 4% massique, plus particulièrement de 0,5% à 3% massique d'un composé (B) ou d'un mélange de composés (B) choisis parmi les composés de formules (B₁₁), (B₁₂), (B₁₃) et (B₁₄).

Selon un autre aspect particulier, la composition (C₂) telle que définie précédemment, est carctérisée en ce que dans la formule (I), ledit reste G d'un sucre réducteur, est choisi parmi les restes du glucose, du dextrose, du saccharose, du fructose, de l'idose, du gulose, du galactose, du maltose, de l'isomaltose, du maltotriose, du lactose, du cellobiose, du mannose, du ribose, du xylose, de l'arabinose, du lyxose, de l'allose, de l'altrose, du dextrane ou du tallose.

Selon un aspect plus particulier, la composition (C₂) telle que définie précédemment est caractérisée en ce que dans la formule (I) ledit reste G d'un sucre réducteur est choisi parmi les restes du glucose, du xylose et de l'arabinose et x représente un nombre décimal supérieur ou égal à 1,05 et inférieur ou égal à 3, plus particulièrement supérieur ou égal à 1,15 et inférieur ou égal à 2,5.

Selon un autre aspect particulier, l'invention a pour objet :
Ou bien la composition (C₂) telle que définie précédemment, pour laquelle, dans la formule (A₁) n est un nombre entier égal à 2, dans la formule (I) n est un nombre entier égal à 2, ledit reste G d'un sucre réducteur représente le reste du glucose, et x représente un nombre décimal compris entre 1,05 et 2,5 et le composé (B), est le composé de formule (B₁₁)
Ou bien la composition (C₂) telle que définie précédemment, pour laquelle, dans la formule (A₁) n est un nombre entier égal à 3, dans la formule (I) n est un nombre entier égal à 3, ledit reste G d'un sucre réducteur représente le reste du glucose, et x représente un nombre décimal compris entre 1,05 et 2,5 et le composé (B) est le composé de formule (B₁₂) ;
Ou bien la composition (C₂) telle que définie précédemment, pour laquelle, dans la formule (A₁) n est un nombre entier égal à 4, dans la formule (I) n est un nombre entier égal à 4, ledit reste G d'un sucre réducteur représente le reste du glucose, et x représente un nombre décimal compris entre 1,05 et 2,5, et le composé (B) est un mélange du composé de formule (B₁₃) et le composé (B₁₄).

La composition (C₂) objet de l'invention peut être obtenue par différentes voies.

Une première voie de préparation de la composition (C₂) consiste, en une première étape E₁), à introduire au moins un polyol de formule (A₁) telle que définie précédemment, au moins une composition (C₁) représentée par la formule (I) telle que définie précédemment, et au moins un composé de formule (B) telle que définie précédemment, dans un réacteur selon un rapport massique maîtrisé, dans des conditions de températures permettant d'assurer l'homogénéité du mélange, préférentiellement entre 60°C et 120°C ; puis si nécessaire ou si désiré, en une seconde étape E₂), à introduire un sucre réducteur de formule (II) telle que définie précédemment au mélange obtenu à l'étape E₁) et à poursuivre jusqu'à l'obtention d'une composition homogène.

Une deuxième voie de préparation de la composition (C₂) consiste, en une première étape E₃), à mettre en oeuvre le procédé de préparation de la composition (C₁), objet de la présente invention, puis si nécessaire ou si désirée, en des étapes ultérieures de neutralisation, de filtration et/ou de décoloration.

Selon un aspect particulier, lorsque la composition (C₂) objet de la présente invention est préparée selon la deuxième voie de préparation, ladite composition (C₂) peut comprendre en outre une quantité résiduelle du sucre réducteur de formule (II) telle que définie précédemment.

Une telle quantité résiduelle de sucre réducteur de formule (II) est comprise, pour 100% de la masse de la composition (C₂), de 0,1% à 10% massique, et plus particulièrement de 0,1% à 4,0% massique.

Les compositions (C₁) et composition (C₂) telles que définies précédemment, peuvent être incorporées dans tout type de formulation cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.).

C'est pourquoi, selon un autre aspect, l'invention a pour objet, l'utilisation de la composition (C₁) directement obtenue par le procédé tel que défini précédemment ou de la composition (C₂) telle que définie précédemment, comme élément constitutif de formulations cosmétiques, dermophamaceutiques ou pharmaceutique à usage topique, dans la préparation des dites formulations cosmétiques, dermophamaceutiques ou pharmaceutique à usage topique.

L'expression "à un usage topique" utilisée dans la définition de la formulation dans laquelle peuvent être incorporées les dites compositions (C₁) ou (C₂), signifie que ladite formulation est mise en oeuvre par application sur la peau, les cheveux, le cuir chevelu ou les muqueuses, qu'il s'agisse d'une application directe dans le cas d'une formulation cosmétique, dermocosmétique, dermo-pharmaceutique ou pharmaceutique ou d'une application indirecte par exemple dans le cas d'un produit d'hygiène corporelle, de soin ou de protection de la peau, se présentant sous la forme d'un article en textile, comme par exemple une lingette, ou en papier, comme par exemple un papier à usage sanitaire.

La formulation cosmétique, dermocosmétique, dermopharmaceutique ou pharmaceutique destinée à un usage topique dans laquelle peuvent être incorporées les dites compositions (C₁) ou (C₂), se présentent notamment sous la forme d'une solution aqueuse ou huileuse, d'une émulsion ou d'une micro-émulsion du type eau dans huile (E/H) ou huile-dans-eau (H/E), d'une émulsion multiple de type eau-dans-huile-dans-eau (E/H/E) ou huile-dans-eau-dans-huile (H/E/H), d'un gel, d'un savon ou d'un syndet, d'un baume, d'une hydro-dispersion, d'une crème, d'une mousse, d'un aérosol ou encore sous forme anhydre comme une poudre.

Ces formulations peuvent être utilisées comme laits nettoyants ou démaquillants, comme lotions nettoyantes ou démaquillantes, comme gels moussants pour le visage ou pour le corps, comme shampooing pour le nettoyage des cheveux et/ou du cuir chevelu, comme après-shampooing pour le traitement des cheveux et/ou du cuir chevelu, comme bain moussant, comme crème, comme lait ou comme lotion pour le soin ou pour la protection du visage, des mains et du corps, comme par exemple comme agent protecteur des rayonnements solaires, comme agent auto-bronzant, comme agent anti-âge, comme agent antirides, comme agent apaisant, comme agent hydratant.

De façon générale ces formulations destinées à un usage topique dans lesquelles peuvent être incorporées les dites compositions (C₁) ou (C₂), comportent également des excipients et/ou des principes actifs habituellement mis en oeuvre dans le domaine des formulations à usage topique, en particulier cosmétiques, dermocosmétiques, pharmaceutiques ou dermopharmaceutiques, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les agents plastifiants, les matières grasses, les huiles et les cires, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les agents répulsifs pour les insectes.

Comme exemples de tensioactifs moussants et/ou détergents, éventuellement présents dans les formulations à usage topique dans lesquelles peuvent être incorporées les dites compositions (C₁) ou (C₂), on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer aux formulations à usage topique dans lesquelles peuvent être incorporées les dites compositions (C₁) ou (C₂), on peut citer les sels de métaux alcalins, les sels de métaux alcalino-terreux, les sels d'ammonium, les sels d'amines, les sels d'amino-alcools d'alkyléthers sulfates, d'alkyl sulfates, d'alkylamidoéther sulfates, d'alkylaryl polyéther sulfates, de monoglycérides sulfates, d'alpha-oléfinesulfonates, de paraffines sulfonates, d'alkyl phosphates, d'alkyléther phosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, d'acides gras.

Parmi les tensioactifs amphotères moussants et/ou détergents que l'on peut associer aux formulations à usage topique dans lesquelles peuvent être incorporées les dites compositions (C₁) ou (C₂), on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents que l'on peut associer aux formulations à usage topique dans lesquelles peuvent être incorporées les dites compositions (C₁) ou (C₂), on peut citer particulièrement les dérivés d'ammoniums quaternaires.

Parmi les tensioactifs non ioniques moussants et/ou détergents que l'on peut associer aux formulations à usage topique dans lesquelles peuvent être incorporées les dites compositions (C₁) ou (C₂), on peut citer plus particulièrement les alkyl-polyglycosides, les dérivés d'huile de ricin, les polysorbates, les amides de coprah, les N-alkylamines.

Parmi les tensioactifs non ioniques moussants et/ou détergents que l'on peut associer aux formulations à usage topique dans lesquelles peuvent être incorporées les dites compositions (C₁) ou (C₂), on peut citer plus particulièrement la composition (C₃) ou un mélange de compositions (C₃), ladite composition (C₃) étant représentée par la formule (IV) :

R₂-O-(G₂)ₚ-H (IV)

Dans laquelle R₂ représente un radical aliphatique linéaire ou ramifié, saturé ou insaturé, comportant de 8 à 14 atomes de carbone, G₂ représente le reste d'un sucre réducteur et p représente un nombre décimal supérieur ou égal à 1,05 et inférieur ou égal à 5, ladite composition (C₃) consistant essentiellement en un mélange de composés représentés par les formules (IV₁), (IV₂), (IV₃), (IV₄) et (IV₅) :

R₂-O-(G₂)₁H (IV₁),

R₂-O-(G₂)₂-H (IV₂),

R₂-O-(G₂)₃-H (IV₃),

R₂-O-(G₂)₄-H (IV₄),

R₂-O-(G₂)₅-H (IV₅),

dans les proportions molaires respectives a₁, a₂, a₃, a₄ et a₅, telles que la somme a₁ + a₂ + a₃ + a₄ + a₅ est égale à 1 et que chacune des proportions a₁, a₂, a₃, a₄ et a₅ est supérieure ou égale à zéro et inférieure ou égale à un.

Comme exemples de tensioactifs épaississants et/ou gélifiants éventuellement présents dans les formulations à usage topique dans lesquelles peuvent être incorporées les dites compositions (C₁) ou (C₂), on peut citer les esters gras d'alkyl-polyglycosides éventuellement alkoxylés, et tout particulièrement les esters de méthylpolyglucoside éthoxylés tels que le PEG 120 méthyl glucose trioléate ou le PEG 120 méthyl glucose dioléate commercialisés respectivement sous les nom GLUCAMATE™LT et GLUMATE™DOE120 ; les esters gras alkoxylés tels que le PEG 150 pentaérythrytyl tétrastéarate commercialisé sous l'nom CROTHIX™DS53, le PEG 55 propylene glycol oléate commercialisé sous l'appellation ANTIL™141 ; les carbamates de polyalkylène glycols à chaînes grasses tels que le PPG 14 laureth isophoryl dicarbamate commercialisé sous l'nom ELFACOS™T211, le PPG 14 palmeth 60 hexyl dicarbamate commercialisé sous l'nom ELFACOS™GT2125.

Comme exemples de tensioactifs émulsionnants éventuellement présents dans les formulations à usage topique dans lesquelles peuvent être incorporées les dites compositions (C₁) ou (C₂), on peut citer des tensioactifs non ioniques, des tensioactifs anioniques, des tensioactifs cationiques.

Comme exemples de tensioactifs non ioniques émulsionnants éventuellement présents dans les formulations à usage topique dans lesquelles peuvent être incorporées les dites compositions (C₁) ou (C₂), on peut citer les esters d'acides gras et de sorbitol, par exemple les MONTANE™80, MONTANE™85 et MONTANE™60 ; les alkylpolyglycosides et les compositions d'alkylpolyglycosides et d'alcools gras linéaires ou ramifiés, saturés ou insaturés, la chaine alkyles desdits alkyl polyglycosides étant constituée par des groupements alkyles linéaires ou ramifiés, saturés ou insaturés, comportant de 14 à 22 atomes de carbone, par exemple les MONTANOV™, EASYNOV™ et FLUIDANOV™ ; les esters d'acide gras et de polyglycérol, par exemple ISOLAN™ GI34 et PLUROL™ DIISOSTEARIQUE ; l'huile de ricin éthoxylée et l'huile de ricin hydrogénée éthoxylée, le SIMULSOL™989 ; les compositions comprenant du stéarate de glycérol et de d'acide stéarique éthoxylé entre 5 moles et 150 moles d'oxyde d'éthylène, par exemple la composition comprenant de l'acide stéarique éthoxylé à 135 moles d'oxyde d'éthylène et du stéarate de glycérol commercialisée sous le nom SIMULSOL™ 165 ; les polyhydroxystéarates de polyglycol ou de polyglycérol par exemple l'HYPERMER™B246 ou l'ARLACEL™P135, le DEHYMULS™PGPH, le DECAGLYN™5HS ; les copolymères polyéthylèneglycol-alkylglycol comme le PEG-45 dodécylglycol copolymère tel que l'ELFACOS™ST 9 ; les esters de sorbitan éthoxylés, par exemple les MONTANOX™ ; les esters de mannitan ; les esters de mannitan éthoxylés ; les esters de sucrose ; les esters de méthylglucoside.

Comme exemples de tensioactifs anioniques émulsionnants éventuellement présents dans les formulations à usage topique dans lesquelles peuvent être incorporées les dites compositions (C₁) ou (C₂), on peut citer le décylphosphate, le cétylphosphate commercialisé sous le nom AMPHISOL™, le glycéryl stéarate citrate ; le cétéarylsulfate ; la composition arachidyl/béhényl phosphates et arachidyl/béhényl alcools commercialisée sous le nom SENSANOV™WR; les savons comme par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans les formulations à usage topique dans lesquelles peuvent être incorporées les dites compositions (C₁) ou (C₂), on peut citer les aminoxydes, le QUATERNIUM™82 et les tensioactifs décrits dans la demande interationale publiée sous le numéro WO 96/00719 et principalement ceux dont la chaîne grasse comprend au moins 16 atomes de carbone.

Comme exemples d'agents opacifiants et/ou nacrants éventuellement présents dans les formulations à usage topique dans lesquelles peuvent être incorporées les dites compositions (C₁) ou (C₂), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans les formulations à usage topique dans lesquelles peuvent être incorporées les dites compositions (C₁) ou (C₂), on peut citer des dérivés N-acylés d'acides aminés, par exemple la lauroyl lysine commercialisée sous le nom AMINOHOPE™LL, l'octenyl starch succinate commercialisé sous le nom DRYFLO™, le myristyl polyglucoside commercialisé sous le nom MONTANOV™14, les fibres de cellulose, les fibres de coton, les fibres de chitosane, le talc, la séricite, le mica.

Comme exemples de solvants et de cosolvants éventuellement présents dans les formulations à usage topique dans lesquelles peuvent être incorporées les dites compositions (C₁) ou (C₂), on peut citer l'eau, les solvants organiques comme le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples d'huiles éventuellement présentes dans les formulations à usage topique dans lesquelles peuvent être incorporées les dites compositions (C₁) ou (C₂), on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les iso-alcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires éventuellement présentes dans les formulations à usage topique dans lesquelles peuvent être incorporées les dites compositions (C₁) ou (C₂), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de matières grasses éventuellement présentes dans les formulations à usage topique dans lesquelles peuvent être incorporées les dites compositions (C₁) ou (C₂), on peut citer les alcools gras saturés ou insaturés, linéaires ou ramifiés, comportant de 8 à 36 atomes de carbone, ou les acides gras saturés ou insaturés, linéaires ou ramifiés, comportant de 8 à 36 atomes de carbone.

Comme exemples de principes actifs éventuellement présents dans les formulations à usage topique dans lesquelles peuvent être incorporées les dites compositions (C₁) ou (C₂), on peut citer :
- Les vitamines et leurs dérives, notamment leurs esters, tels que le rétinol (vitamine A) et ses esters (palmitate de rétinyle par exemple), l'acide ascorbique (vitamine C) et ses esters, les dérives de sucre de l'acide ascorbique (comme par exemple l'ascorbyl glucoside), le tocophérol (vitamine E) et ses esters (comme par exemple l'acétate de tocophérol), les vitamines B3 ou B10 (niacinamide et ses dérivés) ;
- Les composés montrant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ;
- Les composés montrant une action apaisante comme le SEPICALM™S, l'allantoïne et le bisabolol ;
- Les agents anti-inflammatoires ;
- Les composés montrant une action hydratante comme par exemple l'urée, les hydroxyurées, le glycérol, les polyglycérols, le glycérolglucoside, le diglycérolglucoside, les polyglycérylglucosides ;
- Les extraits végétaux riches en polyphénols comme par exemple les extraits de raisin, les extraits de pin, les extraits de vin, les extraits d'olives ;
- Les composés montrant une action amincissante ou lipolytique comme la caféine ou ses dérivés, l'ADIPOSLIM™, l'ADIPOLESS™ ;
- Les protéines N-acylées ; les peptides N-acylés comme par exemple le MATRIXIL™ ; les acides aminés N-acylés ; les hydrolysats partiels de protéines N-acylés ; les acides aminés ; les peptides ; les hydrolysats totaux de protéines ; les extraits de soja, par exemple la RAFFERMINE™ ; les extraits de blé par exemple la TENSINE™ ou la GLIADINE™ ;
- Les extraits végétaux, tels que les extraits végétaux riches en tanins, les extraits végétaux riches en isoflavones ou les extraits végétaux riches en terpènes ;
- Les extraits d'algues d'eau douce ou marines ;
- Les extraits marins en général comme les coraux ;
- Les cires essentielles ; les extraits bactériens ; les céramides ; les phospholipides ;
- Les composés montrant une action antimicrobienne ou une action purifiante, par exemple le LIPACIDE™C8G, le LIPACIDE™UG, le SEPICONTROL™A5 ; l'OCTOPIROX™ ou le SENSIVA™SC50 ;
- Les composés montrant une propriété énergisante ou stimulante comme le PHYSIOGENYL™, le panthénol et ses dérivés comme le SEPICAP™MP ;
   les actifs anti-âge comme le SEPILIFT™DPHP, le LIPACIDE™PVB, le SEPIVINOL™, le SEPIVITAL™, le MANOLIVA™, le PHYTO-AGE™, le TIMECODE™ ; le SURVICODE™ ;
- Les actifs anti-photo vieillissement ;
- Les actifs protecteurs de l'intégrité de la jonction dermo-épidermique ;
- Les actifs augmentant la synthèse des composants de la matrice extracellulaire comme par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (comme par exemple les dérivés de l'acide nicotinique) ou des produits créant une sensation de « fraîcheur » sur la peau (comme par exemple le menthol et des dérivés) ;
- Les actifs améliorant la microcirculation cutanée, par exemple les veinotoniques ; les actifs drainants ; les actifs à visée décongestionnante comme par exemple les extraits de ginko biloba, de lierre, de marron d'inde, de bambou, de ruscus, de petit houx, de *centalla asiatica,* de fucus, de romarin, de saule ;
- Les agents de bronzage ou de brunissement de la peau, comme par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents épaississants et/ou gélifiants éventuellement présents dans les formulations à usage topique dans lesquelles peuvent être incorporées les dites compositions (C₁) ou (C₂), on peut citer par exemple les homopolymères ou copolymères de l'acide acrylique ou de dérivés de l'acide acrylique, les homopolymères ou copolymères de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les galactomannanes comme par exemple la gomme de tara, la gomme de guar, la gomme de fenugrec, la gomme de caroube, la gomme de casse ; les silicates ; la cellulose et ses dérivés ; l'amidon et ses dérivés hydrophiles ; les polyuréthanes.

Comme exemples d'agents épaississants et/ou gélifiants éventuellement présents dans les formulations à usage topique dans lesquelles peuvent être incorporées les dites compositions (C₁) ou (C₂), on peut citer plus particulièrement les polymères de type polyélectrolytes comme par exemple, les copolymères de l'acide acrylique et de l'acide 2-méthyl-[(1-oxo-2-propényl)amino] 1-propane sulfonique (AMPS), les copolymères de l'acrylamide et de l'AMPS, les copolymères de l'AMPS et de l'acrylate de (2-hydroxyéthyle), l'homopolymère de l'AMPS, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés sous les noms SIMULGEL™EG, SEPIGEL™305, SIMULGEL™600, SIMULGEL™NS, SIMULGEL™INS 100, SIMULGEL™FL, SIMULGEL™A, SIMULGEL™SMS 88, SEPINOV™EMT10, SEPIPLUS™400, SEPIPLUS™265, SEPIPLUS™S, SEPIMAX™ZEN.

Comme exemples de filtres solaires éventuellement présents dans les formulations à usage topique dans lesquelles peuvent être incorporées les dites compositions (C₁) ou (C₂), on peut citer tous ceux figurant dans la directive cosmétique 76/768/CEE modifiée annexe VII.

L'étude expérimentale suivante illustre l'invention sans toutefois la limiter.

### A) Exemples de préparation de compositions (C₁) selon le procédé objet de l'invention

### EXEMPLE 1 : Préparation d'une composition (E₁) à base de xylityl glucosides, catalysée par l'acide hypophosphoreux.

On introduit 651,3 grammes de xylitol, soit un équivalent molaire, dans un réacteur à double enveloppe, dans laquelle circule un fluide caloporteur, et muni d'une agitation. Le xylitol est fondu à 100°C. 230,4 grammes de glucose, soit 0,3 équivalent molaire, sont progressivement ajoutés dans le réacteur sous agitation à 100°C.

On ajoute ensuite 7,8 grammes d'acide hypophosphoreux en solution à 50% dans l'eau, soit une proportion massique de 0,9% par rapport à la somme des masses de xylitol et de glucose précédemment introduites.

Le milieu réactionnel est placé sous un vide partiel de 3x10⁴Pa (300mbar) à 1,5x10⁴Pa (150mbar), et maintenu à 100°C-105°C pendant une 5 heures et demi en éliminant l'eau formée au moyen d'un montage de distillation. Le milieu réactionnel est ensuite refroidi à une température de 95°C-100°C et neutralisé par ajout de 5,6 grammes de soude à 48%, pour amener le pH d'une solution à 5% massique dudit mélange à 7,0. On obtient ainsi la composition (E₁).

### EXEMPLE 2 : Préparation d'une composition (E₂) à base de xylityl glucosides, catalysée par l'acide phosphorique.

Le mode opératoire décrit dans l'exemple 1 est mis en oeuvre avec 526,7 grammes de xylitol, soit un équivalent molaire, et 187,3 grammes de glucose, soit 0,3 équivalent molaire en substituant l'acide hypophosphoreux par 1,4 grammes d'acide phosphorique en solution à 75% massique dans l'eau, soit une proportion massique de 0,2% par rapport à la somme des masses de xylitol et de glucose introduites. On obtient ainsi la composition (E₂).

### EXEMPLE 3 : Préparation d'une composition (E₃) à base de sorbityl glucosides catalysée par l'acide hypophosphoreux.

On introduit 499,4 grammes de sorbitol, soit un équivalent molaire, dans un réacteur en verre à double enveloppe, dans laquelle circule un fluide caloporteur, et muni d'une agitation efficace. Le sorbitol est fondu à 105 °C.

987,4 grammes de glucose, soit 2,0 équivalents molaires, sont progressivement ajoutés dans le réacteur sous agitation à 110°C.

On ajoute au mélange ainsi obtenu 2,98 grammes d'acide hypophosphoreux en solution à 50% dans l'eau, soit une proportion massique de 0,2% par rapport à la somme des masses de sorbitol et de glucose précédemment introduites.

Le milieu réactionnel est placé sous un vide partiel de 2x10⁴Pa (200mbar) à 4,6x10³Pa (46mbars), et maintenu à une température de 110°C-130°C pendant 4 heures en éliminant l'eau formée au moyen d'un montage de distillation. Le milieu réactionnel est ensuite refroidi à une température de 95°C-100°C et neutralisé par ajout de 2,7 grammes de soude à 48%, pour amener le pH d'une solution à 5% massique dudit mélange à 7,0. On obtient ainsi la composition (E₃).

### EXEMPLE 4 : Préparation d'une composition (E₄) à base de xylityl glucosides catalysée par l'acide polyphosphorique.

Le mode opératoire décrit dans l'exemple 1 est mis en oeuvre avec 500,2 grammes de xylitol, soit un équivalent molaire, et 177,8 grammes de glucose, soit 0,3 équivalent molaire en substituant l'acide hypophosphoreux par 0,91 grammes d'acide polyphosphorique à 117%, soit une proportion massique de 0,13% par rapport à la somme des masses de xylitol et de glucose introduites. On obtient ainsi la composition (E₄).

### B) Exemples de préparation de compositions selon des procédés de l'état de la technique (exemples comparatifs).

### EXEMPLE A : Préparation d'une composition (E_{A}) à base de xylityl glucosides catalysée par l'acide sulfurique.

Le mode opératoire décrit dans l'exemple 1 est mis en oeuvre avec 621,4 grammes de xylitol, soit un équivalent molaire, et 220,8 grammes de glucose, soit 0,3 équivalent molaire, en remplaçant l'acide hypophosphoreux par 1,7gramme d'acide sulfurique à 98% massique dans l'eau, soit une proportion massique de 0,2% par rapport à la somme des masses de xylitol et de glucose introduites. On obtient la composition (E_{A}).

### EXEMPLE B : Préparation d'une composition (E_{B}) à base de xylityl glucosides catalysée par l'acide méthanesulfonique.

Le mode opératoire décrit dans l'exemple 1 est mis en oeuvre avec 500,6 grammes de xylitol, soit un équivalent molaire, et 177,6 grammes de glucose, soit 0,3 équivalent molaire, en remplaçant l'acide hypophosphoreux par 1,4 gramme d'acide méthanesulfonique, soit une proportion massique de 0,2% par rapport à la somme des masses de xylitol et de glucose introduites. On obtient la composition (E_{B}).

### EXEMPLE C : Préparation d'une composition (E_{C}) à base de xylityl glucosides catalysée par le trifluorure de bore dans l'éther éthylique (BF₃.EtO₂).

Le mode opératoire décrit dans l'exemple 1 est mis en oeuvre avec 585,8 grammes de xylitol, soit un équivalent molaire, et 207,5 grammes de glucose, soit 0,3 équivalent molaire, en remplaçant l'acide hypophosphoreux par 3,9 grammes de trifluorure de bore à 48% massique dans l'éther éthylique (BF₃.EtO₂), soit une proportion massique de 0,5% par rapport à la somme des masses de xylitol et de glucose introduites. On obtient la composition (E_{C}).

### EXEMPLE D : Préparation d'une composition (E_{D}) à base de xylityl glucosides catalysée par un mélange d'acide sulfurique et d'acide hypophosphoreux.

Le mode opératoire décrit dans l'exemple 1 est mis en oeuvre avec 134,3 grammes de xylitol, soit un équivalent molaire, et 52,7 grammes de glucose, soit 0,3 équivalent molaire ; en remplaçant l'acide hypophosphoreux par un mélange de 0,41 grammes d'acide sulfurique à 98% dans l'eau (soit une proportion massique de 0,22% par rapport à la somme des masses de xylitol et de glucose introduites) et de 0,96 grammes d'acide hypophosphoreux à 50% massique dans l'eau (soit une proportion massique de 0,5% par rapport à la somme des masses de xylitol et de glucose introduites). On obtient la composition (E_{D}).

### EXEMPLE E : Préparation d'une composition (E_{E}) à base de sorbityl glucosides selon un procédé de l'état de la technique, catalysée par l'acide sulfurique.

Le mode opératoire décrit dans l'exemple 1 est mis en oeuvre avec 528,1 grammes de sorbitol, soit un équivalent molaire, et 1051,1 grammes de glucose, soit 2,0 équivalent molaire en remplaçant l'acide hypophosphoreux 3,0 grammes d'acide sulfurique à 98 % dans l'eau, soit une proportion massique de 0,2% par rapport à la somme des masses de sorbitol et de glucose introduites. Le mode opératoire mis en oeuvre permet d'obtenir la composition (E_{E}).

### C) Caractérisation des compositions (E₁), (E₂), (E₄) (E_{A}), (E_{B}), (E_{C}) et (E_{D}).

Les compositions (E₁), (E₂) et (E₄) obtenues par le procédé selon l'invention et les compositions (E_{A}), (E_{B}), **(E_{C})** et (E_{D}), obtenues selon des procédés de l'état de la technique, ont été analysées pour déterminer la teneur massique des différents composés les constituant, au moyen d'un chromatographe en phase gazeuse, munie d'une colonne métallique HT-SIMDIST™ CB (PE Chropack™) 10 m x 0,53 mm ID-épaisseur de film à 0,5 µm, avec l'hélium comme gaz vecteur, et équipé d'un détecteur de type FID. Les résultats obtenus sont consignés dans le Tableau 1 ci-dessous.

**Tableau 1**

| | (E₁) | (E₂) | (E₄) | (E_{A}) | (E_{B}) | (E_{C}) | (E_{D}) |
|---|---|---|---|---|---|---|---|
| Xylitol | 59,1% | 57,1% | 57,6% | 26,0% | 33,3% | 43,7% | 17,7% |
| 1,4 anhydroxylitol⁽¹⁾ | 2,4% | 0,6% | 1,2% | 32,2% | 22,9% | 17,2% | 37,6% |
| Xylityl glucosides | 38,5% | 42,3% | 41,2% | 41,8% | 43,8% | 39,1% | 44,7% |
| Degré moyen de polymérisation (x) | 1,26 | 1,26 | 1,26 | 1,46 | 1,32 | 1,29 | 1,42 |

| | | | | | | | |
|---|---|---|---|---|---|---|---|
| (1) : le 1,4-anhydroxylitol correspond au produit de déshydratation du xylitol de formule (B₁₂). | | | | | | | |

### D) Caractérisation des compositions (E₃) et (E_{E}).

La composition (E₃) obtenue par le procédé selon l'invention et la composition (E_{E}) obtenue selon un procédé de l'état de la technique, ont été analysées pour déterminer la teneur massique des différents composés les constituant, au moyen d'un chromatographe en phase gazeuse, munie d'une colonne métallique HT-SIMDIST™ CB (PE Chropack™) 10 m x 0,53 mm ID-épaisseur de film à 0,5 µm, avec l'hélium comme gaz vecteur, et équipé d'un détecteur de type FID. Les résultats obtenus sont consignés dans le tableau 2 ci-dessous.

**Tableau 2**

| | (E₃) | (E_{E}) |
|---|---|---|
| Sorbitol | 10,1% | 0,1% |
| sorbitan⁽²⁾ | 1,7% | 16,9% |
| isosorbide⁽³⁾ | 0,1% | 14,4% |
| Sorbityl glucosides | 88,1% | 68,6% |
| Degré moyen de polymérisation (x) | 2,15 | 2,10 |

| | | |
|---|---|---|
| (2) : le 1,4-anhydrosorbitol correspond au produit de déshydratation du sorbitol de formule (B₁₃). (3) : l'isosorbide correspond au produit de déshydratation du 1,4-anhydrosorbitol de formule (B₁₄). | | |

### E) Analyse et commentaires des résultats.

Les analyses réalisées pour les compositions (E₁), (E₂) et (E₄), résultant de la mise en oeuvre du procédé selon l'invention et impliquant respectivement l'acide hypophosphoreux, l'acide phosphorique et l'acide polyphosphorique comme catalyseur de réaction, montrent une teneur en 1,4 anhydroxylitol, substance de formule (B₁₂) telle que définie précédemment, résultant de la déshydratation du xylitol, respectivement de 2,4% pour la composition (E₁), de 0,6% pour la composition (E₂) et de 1,2% pour la composition (E₄). Les compositions (E_{A}), (E_{B}), (Ec) et (E_{D}) se caractérisent par des teneurs en 1,4-anhydroxylitol, respectivement égales à 32,2%, 22,9%, 17,2% et 37,6%.

Les analyses réalisées montrent que la composition (E₃), résultant de la mise en oeuvre du procédé selon l'invention impliquant l'acide hypophosphoreux comme catalyseur de réaction, se caractérise par une teneur totale en espèces déshydratées du sorbitol de 1,8%, alors que la composition (E_{E}), obtenue par la mise en oeuvre d'un procédé impliquant l'acide sulfurique comme catalyseur de réaction, contient une teneur totale en espèces déshydratées du sorbitol égale à 31,3%.

Les procédés selon l'invention, mettant en oeuvre un catalyseur acide choisi parmi l'acide hypophosphoreux, l'acide phosphorique et l'acide polyphosphorique lors de la réaction d'acétalisation d'un sucre réducteur avec un polyol de formule (A₁) telle que définie précédemment, permettent donc de préparer des compositions à base de polyol-glycosides de formule (I) avec une quantité restreinte de sous-produits de déshydratation du polyol de formule (A₁).

### F) Formulations

Dans les formules suivantes, les pourcentages sont exprimés en poids de la formulation.

### F.1 Fluide démaquillant visage

**Formule**

| | |
|---|---|
| Composition (E₁) | 10,00% |
| Méthyl paraben | 0,15% |
| Phenoxyethanol | 0,80% |
| SEPICALM™ S | 1,00% |
| Parfum/Fragrance | 0,10% |
| Eau | qs. 100,00% |

Mode opératoire : Mélanger les différents ingrédients dans l'eau sous agitation magnétique dans l'ordre indiqué, et ajuster le pH aux alentours de 7.

### F.2 Shampoing cheveux et corps pour enfants

**Formule**

| | | |
|---|---|---|
| A | Composition (E₁) | 15,00% |
| | PROTEOL™APL | 5,00% |
| | SEPICIDE™HB | 0,50% |
| | Parfum/Fragrance | 0,10% |
| B | Eau | 20,00% |
| | CAPIGEL™98 | 3,50% |
| C | Eau | Q.S. 100,00% |
| | SEPICIDE™Cl | 0,30% |
| | Colorant | Q.S |
| | Soude | Q.S. pH = 7,2 |

Mode opératoire : Mélanger la composition (E₁) avec le PROTEOL™APL, et le SEPICIDE™HB (Phase A). Diluer le CAPIGEL™98 dans une partie de l'eau et l'ajouter à la phase A précédemment obtenue (Phase B). Ajouter le reste d'eau à la phase B, puis le SEPICIDE™Cl et le colorant. Ajuster le pH du mélange à 7,2 environ avec de la soude.

### F.3 Lingettes démaquillantes pour les yeux

**Formule**

| | | |
|---|---|---|
| A | Composition (E₁) | 3,00% |
| B | SEPICIDE™HB2 | 0,50% |
| C | SEPICALM™ VG | 0,50% |
| | Glycérine | 10,00% |
| | Parfum/Fragrance | 0,05% |
| D | Eau | Q.S. 100,00% |

Mode opératoire : Mélanger les ingrédients de la phase B ainsi que ceux de la phase C dans la phase A jusqu'à obtenir la limpidité de la solution. Ajouter la phase D .

### F.4 Gel moussant doux

**Formule**

| | | |
|---|---|---|
| A | Composition (E₂) | 8,50% |
| | PROTEOL™ APL | 3,00% |
| | EUXYL ™ PE9010 | 1,00% |
| | Parfum/Fragrance | 0,10% |
| B | Eau | Q.S. 100,00% |
| | Acide lactique | Q.S. pH = 6,0 |

Mode opératoire : Solubiliser le parfum et le conservateur EUXYL™ PE 9010 dans le mélange composé de la composition E₂ et du PROTEOL™ APL (phase A). Ajouter l'eau et régler le pH à environ 6,0 avec de l'acide lactique.

### F.5 Shampoing à usage fréquent

**Formule**

| | | |
|---|---|---|
| A | Composition (E₂) | 12,80% |
| | PROTEOL™ OAT | 5,00% |
| | EUXYL ™ PE 9010 | 1,00% |
| | Parfum/Fragrance | 0,30% |
| | Eau | Q.S. 100,00% |
| B | MONTALINE™C40 | 8,50% |
| | Acide lactique | Q.S. pH = 6,0 |

Mode opératoire : Mélanger tous les ingrédients de la phase A et, après homogénéisation, ajouter la MONTALINE™C40 et ajuster le pH à environ 6,0 à l'aide de l'acide lactique.

### F.6 Shampoing ultra-doux pour bébé

**Formule**

| | | |
|---|---|---|
| A | Composition (E₃) | 10,00% |
| | AMISOFT™CS-11 | 4,00% |
| | Parfum/Fragrance | 0,10% |
| | SEPICIDE™HB | 0,30% |
| | SEPICIDE™Cl | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : Mélanger tous les ingrédients de la phase A dans l'ordre indiqué jusqu'à l'obtention d'une phase A limpide. De façon séparée, ajouter le CAPIGEL™98 dans l'eau, puis ajouter cette phase B ainsi préparée dans la phase A et ajuster le pH à 7,2 à l'aide de la trométhamine.

### F.7 Lait de toilette pour bébé

**Formule**

| | | |
|---|---|---|
| A | SIMULSOL™165 | 2,00% |
| | MONTANOV™202 | 1,00% |
| | LANOL™99 | 3,00% |
| | Dimethicone | 1,00% |
| | Isohexadecane | 3,00% |
| B | Eau | Q.S. 100,00% |
| C | SEPIPLUS™400 | 0,30% |
| D | Composition (E₁) | 6,35% |
| E | SEPICIDE™HB | 0,30% |
| | DMDM Hydantoin | 0,20% |
| | Parfum/Fragrance | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

### F.8 Lotion poudrée nettoyante pour peaux sensibles

**Formule**

| | | |
|---|---|---|
| A | LIPACIDE™C8G | 0,95% |
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | Composition (E₂) | 1,80% |
| | Parfum/Fragrance | 0,10% |
| C | SEPICALM™S | 0,28% |
| | Eau | Q.S. 100,00% |
| | Acide lactique | Q.S. pH = 5,2 |
| D | MICROPEARL™M310 | 5,00% |

Mode opératoire : Solubiliser les ingrédients de la phase A dans l'eau à 80°C. Solubiliser séparément le parfum dans la composition (E₂) pour préparer la phase B. Ajouter la phase A refroidie sur la phase B, puis introduire le SEPICALM™S et le complément d'eau. Vérifier le pH final et éventuellement l'ajuster à environ 5,2. Ajouter alors le MICROPEARL™ M310.

### F.9 Gel douche enfants

**Formule**

| | | |
|---|---|---|
| A | Eau | 56,06% |
| | SEPIMAX™Zen | 3,00% |
| | SEPIPLUS™S | 0,80% |
| B | PROTEOL™OAT | 20,80% |
| | ORAMIX™NS 10 | 9,30% |
| | AMONYL™265 BA | 5,10% |
| C | Composition (E₁) | 2,00% |
| Glycéryl Glucoside | | 1,00% |
| Phenoxyéthanol & Ethylhexyl Glycérine | | 1,00% |
| Parfum/Fragrance | | 0,90% |
| Colorant | | 0,04% |

Mode opératoire : disperser le SEPIMAX™ZEN dans l'eau et agiter à l'aide d'un agitateur mécanique muni d'une défloculeuse, d'une contrehélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogéniser et ajouter individuellement les additifs de la phase C. Ajuster le pH à 6.0 - 6.5.

### F.10 BB Crème

**Formule**

| | | |
|---|---|---|
| A | EASYNOV™ | 2,30% |
| | LANOL™ 99 | 1,00% |
| | SEPIMAT™ H10W | 1,00% |
| | Ethylhexyl methoxycinnamate | 5,00% |
| B | Cyclométhicone | 6,00% |
| | Triethoxycaprylsilane & Alumina-silane & Titanium Oxide | 8,00% |
| | Iron Oxide red & Triethoxycaprylsilane | 0,24% |
| | Iron Oxide yellow & Triethoxycaprylsilane | 0,66% |
| | Iron Oxide black & Triethoxycaprylsilane | 0,09% |
| | Parfum/Fragrance | 0,10% |
| C | Eau | qs 100% |
| | Glycérol | 6,00% |
| | SEPINOV™EMT10 | 1,20% |
| D | Composition (E₁) | 2,00% |
| | SEPITONIC™M3 | 1,00% |
| | Phenoxyéthanol & Ethylhexyl Glycérine | 1,00% |

Mode opératoire : Préparer la phase B par mélange des différents ingrédients et homogénéiser à l'aide d'un mélangeur muni d'un système de rotor-sator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-sator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase 5 et agiter à une vitesse de 50 tours par minute pendant 25 minutes.

### F.11 Spray Solaire haute Protection SPH supérieur à 30

**Formule**

| | | |
|---|---|---|
| A | MONTANOV™L | 1,00% |
| | MONTANOV™82 | 1,00% |
| | C12-15 Alkylbenzoate | 17,00% |
| | Dimethicone | 3,00% |
| | Octocrylène | 6,00% |
| | Ethylhexyl methoxycinnamate | 6,00% |
| | Bis-ethylhexyloxyphenol Méthoxypenyl Triazine | 3,00% |
| | Tocophérol | 0,05% |
| B | Eau | qs 100% |
| C | SIMULGEL™INS 100 | 0,50% |
| | Cyclodiméthicone | 5,00% |
| D | Composition (E₁) | 3,00% |
| | Phenoxyéthanol & Ethylhexyl Glycérine | 1,00% |
| | Parfum/Fragrance | 0,20% |
| E | Methylene Bis-Benzotriazolyl | |
| | Tetraméthylbutylphénol | 10,00% |
| | Acide citrique 25% | qs pH = 5 |

SEPICALM™S : Mélange de N-cocoyl aminoacides, de sarcosine, d'aspartate de potassium et d'aspartate de magnésium tel que décrit dans WO 98/09611 et commercialisé par la société SEPPIC.
PROTEOL™APL : Mélange de sels de sodium de N-cocoyl aminoacides, obtenus par acylation des acides aminés caractéristiques du jus de pomme et commercialisé par la société SEPPIC.
SEPICIDE™HB, un mélange de phénoxyéthanol, de méthylparaben, d'éthylparaben, de propylparaben et de butylparaben, est un agent conservateur commercialisé par la société SEPPIC.
CAPIGEL™98 est un copolymère d'acrylates commercialisé par la société SEPPIC.
SEPICIDE™Cl, imidazoline urée, est un agent conservateur commercialisé par la société SEPPIC.
SEPICIDE™HB est un mélange de phénoxyéthanol, de méthylparaben, d'éthylparaben, de propylparaben, de butylparaben et d'isobutylparaben, est un agent conservateur commercialisé par la société SEPPIC.
SEPICALM™VG est un mélange de N-palmitoyl proline sous forme de sel de sodium et d'extrait de fleur de Nymphea Alba, commercialisé par la société SEPPIC.
EUXYL™PE9010, mélange de phénoxyéthanol et d'Ethyl Hexyl Glycérine, est un agent conservateur commercialisé par la société SEPPIC.
PROTEOL™OAT est un mélange de N-lauryl aminoacides obtenus par hydrolyse totale de protéine d'avoine tel que décrit dans WO 94/26694 et commercialisé par la société SEPPIC.
MONTALINE™C40 est un sel de chlorure de Cocamidopropyl betaïnamide de Monoéthanolamine.
AMISOFT™CS-11 est un sel de sodium de N-cocoyl glutamate, commercialisé par la société AJINOMOTO.
SIMULSOL™165 est un mélange de stéaraate de PEG-100 et de stéarate de glycérol, commercialisé par la société SEPPIC.
MONTANOV™202 (alcool arachydilique, alcool béhénique et arachidyl glucoside), est une composition auto-émulsionnable telle que celles décrites dans EP 0 977 626, commercialisée par la société SEPPIC.
LANOL™99 est l'isononoate d'isononyle commercialisé par la société SEPPIC.
SEPIPLUS™400 est un latex inverse auto-inversible de polyacrylates dans le polyisobutene et comportant du polysorbate 20, tel que décrit dans WO2005/040230 et commercialisé par la société SEPPIC.
LIPACIDE™C8G est du capryloyl glycine commercialisé par la société SEPPIC.
MICROPEARL™M310 est un polymère polyméthylméthacrylate réticulé se présentant sous forme de poudre et utilisé comme modificateur de texture.
SEPIMAX™Zen (nom INCI : Polyacrylate Crosspolymer-6) est un polymère épaississant se présentant sous la forme d'une poudre et commercialisé par la société SEPPIC.
SEPIPLUS™S (nom INCI : Hydroxyethyl Acrylate / Sodium Acryloyldimethyl Taurate Copolymer & Polyisobutene & PEG-7 Trimethylolpropane Cononut Ether) est un latex inverse auto-inversible utilisé comme agent épaississant et commercialisé par la société SEPPIC.
AMONYL™265 BA (nom INCI : Cocobétaïne) est un agent tensioactif amphotère moussant et commercialisé par la société SEPPIC.
SEPINOV™EMT10 (nom INCI : Hydroxyethyl Acrylate / Sodium Acryloyldimethyl Taurate Copolymer) est un copolymère épaississant se présentant sous la forme d'une poudre et commercialisé par la société SEPPIC.
EASYNOV™ (nom INCI: Octyldodecanol and Octyldodecyl Xyloside and PEG-30 Dipolyhydroxystearate) est un agent émulsionnant à tendance lipophile commercialisé par la société SEPPIC.
SEPIMAT™H10 FW (nom INCI :Methyl Methacrylate Crosspolymer and Squalane) est un polymère utilisé comme agent de texture et commercialisé par la société SEPPIC.
SEPITONIC™M3 (nom INCI : Magnesium Aspartate and Zinc Gluconate and Copper Gluconate) est un mélange utilisé comme ingrédient énergisant pour les cellules et comme agent anti-radicalaire.
MONTANOV™L (nom INCI : C14-22 Alcohols and C12-20 Alkylglucoside) est un agent émulsionnant commercialisé par la société SEPPIC.
MONTANOV™82 (nom INCI : Cetearyl Alcohol and Coco-glucoside) est un agent émulsionnant commercialisé par la société SEPPIC.
SIMULGEL™INS100 (nom INCI : Hydroxyethyl Acrylate/Sodium Acryloydimethyl Taurate Copolymer and isohexadecane and Polysorbate 60) est un agent épaississant polymèrique se présentant sous la forme d'un latex inverse et commercialisé par la société SEPPIC.

## Revendications

1. Composition (C₂) comprenant pour 100% de sa masse :
- De 1% à 70% massique d'un polyol de formule (A₁) :
HO-CH₂-(CHOH)ₙ-CH₂-OH (A₁)
dans laquelle n est un nombre entier égal à 2, 3 ou 4 ;
- De 25% à 98,9% massique d'une composition (C₁) représentée par la formule (I) :
HO-CH₂-(CHOH)ₙ-CH₂-O-(G)ₓ-H (I),
Dans laquelle G représente le reste d'un sucre réducteur, n est un nombre entier égal à 2, 3 ou 4 et x, qui indique le degré moyen de polymérisation dudit reste G, représente un nombre décimal supérieur à 1 et inférieur ou égal à 5 ;
- De 0,1% à 5% massique d'un composé (B) ou d'un mélange de composés (B) choisis parmi :
- Le composé de formule (B₁₁) : 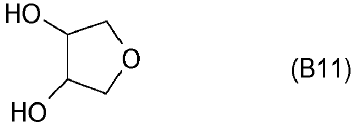
- Le composé de formule (B₁₂) : 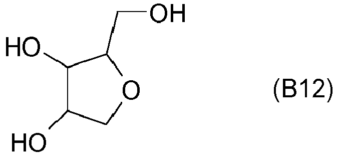
- Le composé de formule (B₁₃) : 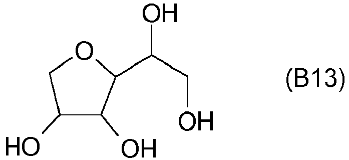
- Et le composé de formule (B₁₄) :

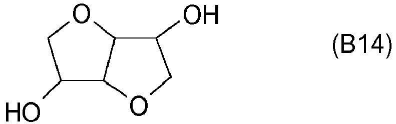

2. Composition (C₂) telle que définie à la revendication 1 **caractérisée en ce qu'**elle comprend pour 100% de sa masse :
- De 5% à 65% massique, plus particulièrement de 10% à 60% massique d'au moins un polyol de formule (A₁),
- De 31% à 94,5% massique, plus particulièrement de 37% à 89,5% massique d'au moins une composition (C₁),
- De 0,5% à 4% massique, plus particulièrement de 0,5% à 3% massique d'un composé (B) ou d'un mélange de composés (B) choisis parmi les composés de formules (B₁₁), (B₁₂), (B₁₃) et (B₁₄).

3. Composition (C₂) telle que définie telle que définie à l'une quelconque des revendications 1 ou 2, **caractérisée en ce que** dans la formule (I), ledit reste G d'un sucre réducteur, est choisi parmi les restes du glucose, du dextrose, du saccharose, du fructose, de l'idose, du gulose, du galactose, du maltose, de l'isomaltose, du maltotriose, du lactose, du cellobiose, du mannose, du ribose, du xylose, de l'arabinose, du lyxose, de l'allose, de l'altrose, du dextrane ou du tallose.

4. Composition (C₂) telle que définie telle que définie à la revendication 3, **caractérisée en ce que** dans la formule (I) ledit reste G d'un sucre réducteur est choisi parmi les restes du glucose, du xylose et de l'arabinose et x représente un nombre décimal supérieur ou égal à 1,05 et inférieur ou égal à 3.

5. Composition (C₂) telle que définie telle que définie à la revendication 4, **caractérisée en ce que** dans la formule (I) ledit reste G d'un sucre réducteur est choisi parmi les restes du glucose, du xylose et de l'arabinose et x représente un nombre décimal supérieur ou égal à 1,15 et inférieur ou égal à 2,5.

6. Composition (C₂) telle que définie à la revendication 5, pour laquelle, dans la formule (A₁) n est un nombre entier égal à 2, dans la formule (I) n est un nombre entier égal à 2, ledit reste G d'un sucre réducteur représente le reste du glucose et x représente un nombre décimal compris entre 1,05 et 2,5 et le composé (B), est le composé de formule (B₁₁).

7. Composition (C₂) telle que définie à la revendciation 5, pour laquelle, dans la formule (A₁) n est un nombre entier égal à 3, dans la formule (I) n est un nombre entier égal à 3, ledit reste G d'un sucre réducteur représente le reste du glucose, et x représente un nombre décimal compris entre 1,05 et 2,5 et le composé (B) est le composé de formule (B₁₂).

8. Composition (C₂) telle que définie à la renvendication 5, pour laquelle, dans la formule (A₁) n est un nombre entier égal à 4, dans la formule (I) n est un nombre entier égal à 4, ledit reste G d'un sucre réducteur représente le reste du glucose, et x représente un nombre décimal compris entre 1,05 et 2,5, et le composé (B) est un mélange du composé de formule (B₁₃) et le composé (B₁₄).

9. Utilisation de la composition (C₂) telle que définie à l'une des revendications 1 à 8, comme élément constitutif de formulations cosmétiques, dermophamaceutiques ou pharmaceutique à usage topique, dans la préparation des dites formulations cosmétiques, dermophamaceutiques ou pharmaceutiques à usage topique.
